(19) Europäisches Patentamt

European Patent Office

Office européen des brevets

(11) Veröffentlichungsnummer: **0 377 791 B1**

## (12) EUROPÄISCHE PATENTSCHRIFT

(45) Veröffentlichungstag der Patentschrift: **22.11.95**

(51) Int. Cl.⁶: **G01N 33/28**

(21) Anmeldenummer: **89117834.5**

(22) Anmeldetag: **27.09.89**

Die Akte enthält technische Angaben, die nach
dem Eingang der Anmeldung eingereicht wurden
und die nicht in dieser Patentschrift enthalten sind.

(54) **Verfahren zur Feststellung des Alkoholgehaltes und/oder des Heizwertes von Kraftstoffen.**

(30) Priorität: **09.12.88 DE 3841471**

(43) Veröffentlichungstag der Anmeldung:
**18.07.90 Patentblatt 90/29**

(45) Bekanntmachung des Hinweises auf die
Patenterteilung:
**22.11.95 Patentblatt 95/47**

(84) Benannte Vertragsstaaten:
**DE FR GB IT SE**

(56) Entgegenhaltungen:

| | |
|---|---|
| **EP-A- 0 069 969** | **DE-A- 3 412 704** |
| **FR-A- 2 177 338** | **GB-A- 1 006 523** |
| **US-A- 3 965 416** | **US-A- 4 764 718** |

**PATENT ABSTRACTS OF JAPAN, Band 7, Nr.
197 (M-239)[1342], 27. August 1983/**

(73) Patentinhaber: **FEV Motorentechnik GmbH &
Co. KG
Neuenhofstrasse 181
D-52078 Aachen (DE)**

(72) Erfinder: **Schmitz, Günter, Dr.-Ing.
Zehnthofweg 31
D-5100 Aachen (DE)**

(74) Vertreter: **Langmaack, Jürgen, Dipl.-Ing. et al
Patentanwälte
Maxton . Maxton . Langmaack
Postfach 51 08 06
D-50944 Köln (DE)**

**Beschreibung**

In Anbetracht der langfristig nur in begrenztem Umfang verfügbaren Reserven an fossiler Energie, insbesondere der aus Rohöl gewonnenen Kraftstoffe, und in Anbetracht der steigenden Anforderungen an den Umweltschutz, wird diesen Kraftstoffen in zunehmendem Maße Methyl- oder Äthylalkohol zugemischt. Dabei soll ein beliebiges Tanken sowohl von Reinkraftstoffen als auch von Mischkraftstoffen möglich sein. Bei höheren Alkoholanteilen ist dabei die Kenntnis des Mischungsverhältnisses erforderlich, um eine optimale Arbeitsweise der Brennkraftmaschine zu erreichen und dabei insbesondere eine genaue, den Betriebsverhältnissen angepaßte Kraftstoffzumessung zu ermöglichen. Besondere Probleme bereitet dabei die laufende Feststellung des Alkoholgehaltes des der Brennkraftmaschine im Betrieb laufend zugeführten Kraftstoffs bei Fahrzeugmotoren, bei denen durch das beliebige Tanken der Kraftstoffarten jede mögliche Mischung erreicht werden kann.

Die bekannten optischen Verfahren sind zu diesem Zweck kaum geeignet, da sie meist Grenzflächeneffekte zur Bestimmung des Brechungsindexes ausnutzen, aus dem dann auf den Alkoholanteil geschlossen werden kann. Abgesehen von der Schwierigkeit der Auswertung bei Fahrzeugmotoren ist ein Nachteil dieses Verfahrens auch, daß die messend zu beobachtende Mischung eine hohe Homogenität aufweisen muß, die insbesondere auch an der Grenzfläche vorhanden sein muß. Mit diesem Verfahren wurden nicht die erforderlichen Genauigkeiten erreicht.

Es wurde daher vorgeschlagen, den Alkoholanteil in Kraftstoffen durch eine Dielektrizitätsbestimmung festzustellen, und ein solches Verfahren hätte den Vorteil, daß die Problematik der Messung von Grenzflächeneffekten behoben ist, da die Messung volumetrisch erfolgt. Andererseits geht in die volumetrische Dielektrizitätsbestimmung in starkem Maße der Leitwert der Mischung ein (Querempfindlichkeit). Da aber der Leitwert sehr stark von Verunreinigungen oder Wasseranteilen abhängt, führt auch ein solches Meßverfahren zu unbrauchbaren Ergebnissen.

In der Schrift "Proceedings of the Fourth International Symposium on Alcohol Fuels Technology", Sao Paulo, Brasilien, vom 05.10.1980, wird die Möglichkeit der Feststellung des Alkoholgehaltes von Kraftstoffen durch Dielektrizitätsmessungen beschrieben. Aufgrund der Einflüsse von Temperatur und Leitwert (hervorgerufen durch Wasseranteile oder andere Verschmutzungen im Kraftstoff) wurde das Verfahren jedoch verworfen, da eine für Verbrennungsmotoren geeignete, zuverlässige Messung nicht durchgeführt werden konnte.

In der EP-A-0 069 969 wird ein Verfahren zur Bestimmung des Alkoholgehaltes in einem Kraftstoff angegeben, bei dem die Leitfähigkeit des zu untersuchenden Stoffgemisches gemessen wird, wobei die Messung der Änderung des Spannungsabfalles in Anhängigkeit von der mengenmäßigen Alkoholbeimischung erfaßt wird. Eine erhöhte Leitfähigkeit entspricht einer erhöhten Dielektrizitätszeit. Sind jedoch in einem Kraftstoff und zwar sowohl in dem beigemischten Alkohol als auch in dem Grundkraftstoff Verunreinigungen vorhanden, dann ergibt eine derartige Leitfähigkeitsmessung einen Alkoholanteil, der mit dem tatsächlich beigemischten Alkoholanteil nicht übereinstimmt, so daß sich eine Fehlmessung ergibt.

In der DE-A-34 12 704 ergibt sich ein vergleichbarer Effekt. Durch das Vorhandensein von Verunreinigungen und der hierdurch erhöhten Leitfähigkeit erhöht sich auch die Dämpfung des Gemisches. Die "Messung" führt daher wiederum zu einem Meßergebnis, das einen Alkohol anzeigt, der über dem tatsächlichen Alkoholgehalt liegt.

In dem Verfahren nach Patent abstract of Japan 7(197)(M-239)(1342) ergibt sich durch etwa enthaltene Verunreinigungen und eine dadurch erhöhte Leitfähigkeit neben dem Abflachen der Resonanzkurve auch eine Verschiebung der Resonanzfrequenz zu tiefen Frequenzen hin. Somit erfolgt auch hier wieder eine Fehlmessung im Sinne eines zu hohen Alkoholgehaltes. Die hier beschriebene Resonanzfrequenzmessung ist schaltungstechnisch sehr aufwendig, da hier jeweils die Resonanzfrequenz mit Hilfe eines Durchstimmens des Resonators mitMaiximumsuche bestimmt werden muß.

Der Erfindung liegt daher die Aufgabe zugrunde, ein Verfahren zu schaffen, das insbesondere in der Anwendung auf Fahrzeugmotoren auch bei Verunreinigungen im Kraftstoff eine genaue und zuverlässige Feststellung des Alkoholgehaltes und/oder des Heizwertes von Kraftstoffen erlaubt.

Gemäß der Erfindung wird diese Aufgabe mit dem im Anspruch 1 augegebenen Verfahren gelöst.

Der besondere Vorteil des erfindungsgemäßen Verfahrens besteht darin, daß durch die Auswahl eines zeitbezogenen Parameters für die Durchführung der Messung der Einfluß von Verunreinigungen weitgehend eliminiert wird. Durch Verunreinigungen bewirkte unterschiedliche Leitfähigkeit haben auf die zeitbezogenen Ausbreitungsparameter einer elektro-magnetischen Welle nahezu keinen Einfluß.

Gemäß einer bevorzugten Ausführungsform ist vorgesehen, daß in einem mit Kraftstoff gefüllten Wellenleiter die Laufzeit oder die Laufzeitdifferenz einer elektromagnetischen Welle zwischen zwei oder mehr Punkten bestimmt und rechnerisch oder schaltungstechnisch als Maß für den Alkoholgehalt und/oder den Heizwert ausgewertet wird.

Auch kann es vorteilhaft sein, daß die Laufzeit der reflektierten Welle als Maß für den Alkoholgehalt und/oder den Heizwert ausgewertet wird.

Um eine höhere Genauigkeit der Messung zu erzielen und/oder einen Einfluß von Verunreinigungen oder Zusätzen des Kraftstoffs zumindest teilweise zu kompensieren, ist es zweckmäßig, mehrere der Ausbreitungsparameter zu bestimmen und in Kombination auszuwerten.

Um Vorteile hinsichtlich der Störunempfindlichkeit gegenüber Temperatureinflüssen und elektromagnetischer Einstrahlung, Baugröße, Kontaktierung, Leitungsführung, Störabstrahlung o.dgl. zu erreichen, kann die Meßschaltung und gegebenenfalls auch die Auswerteschaltung ganz oder teilweise in den Wellenleiter oder Resonator integriert werden.

Hinsichtlich weiterer bevorzugter Ausführungsformen wird auf die Unteransprüche und die nachfolgende Beschreibung von Ausführungsbeispielen Bezug genommen.

Ausbreitungsparameter sind beispielsweise Phasengeschwindigkeit, Phasenmaß, Dämpfungsmaß, Wellenwiderstand, Dispersion und Leitungswellenlänge. Alle diese Größen hängen von Stoffparametern der Kraftstoffe ab, mit denen die Hochfrequenzleitung bzw. der Resonator gefüllt ist.

Insbesondere besteht eine Abhängigkeit dieser Größen von der Dielektrizitätskonstanten, die wiederum vom verwendeten Mischungsverhältnis des Kraftstoffs abhängt. Weiterhin geht auch die Leitfähigkeit in diese Ausbreitungsparameter ein, die auch von dem Mischungsverhältnis abhängt, jedoch auch durch Zusätze oder Verunreinigungen im Kraftstoff beeinflußt wird.

Der Vorteil des Verfahrens gemäß der Erfindung besteht insbesondere in der weitgehenden Unabhängigkeit der genannten Ausbreitungsparameter von geometrischen Größen, so daß Fertigungstoleranzen kaum eine Rolle spielen. Dies hat eine kostengünstigere Herstellung zur Folge, da Abgleichprozeduren zum Ausgleich von Fertigungstoleranzen entfallen können und auch größere Toleranzen akzeptabel sind.

Ausführungsbeispiele der Erfindung werden anhand der Zeichnungen beschrieben.

Fig. 1 zeigt im Diagramm die Dielektrizitätszahl als Funktion des Leitwertes G in Abhängigkeit von dem Wassergehalt und dem Methanolgehalt des Kraftstoffs.

Fig. 2 zeigt schematisch eine Schaltung zur Feststellung der Ausbreitungsgeschwindigkeit durch Messung der Laufzeit oder der Laufzeitdifferenz zwischen zwei Punkten.

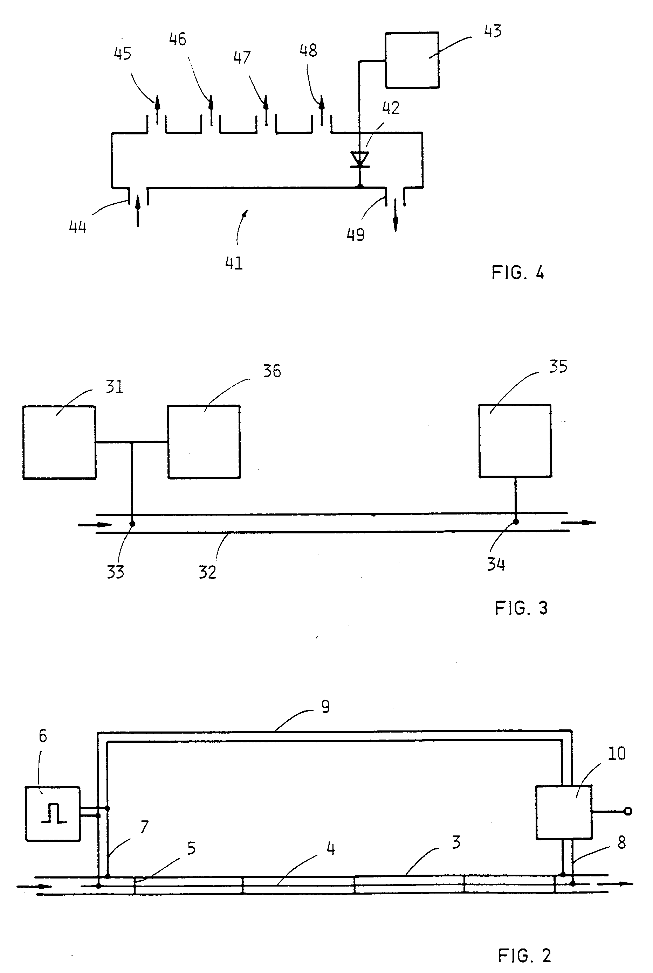

Anhand der Fig. 3 werden zwei bevorzugte Ausführungsformen zur Bestimmung der Cut-Off-Frequenz beschrieben.

Fig. 4 zeigt als Ausführungsbeispiel die Anwendung des Verfahrens gemäß der Erfindung bei einem Teil des Kraftstoffsystems einer Brennkraftmaschine.

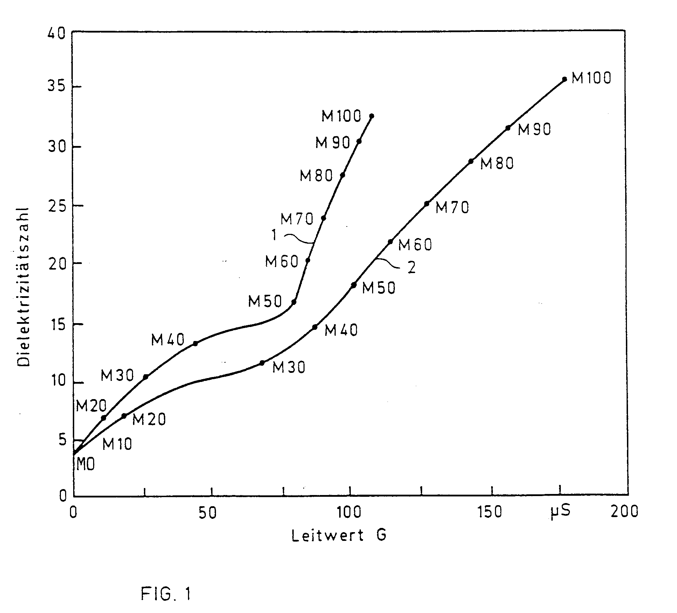

In der Darstellung gemäß Fig. 1 sind die Werte der Dielektrizitätsmessung in der Ordinate und die Leitwerte G in der Abszisse aufgetragen. Linienzug 1 zeigt die Abhängigkeit der Werte der Dielektrizitätsmessung von dem Anteil der Methanolbeimischung bei einem Wasseranteil von 0% im Kraftstoff, während Linienzug 2 die entsprechenden Werte bei einem Anteil von 2,5% $H_2O$ darstellt. Auf den Linienzügen sind die jeweiligen Meßpunkte für variable Methanolanteile von 0% bis 100% (MO bis M100) aufgetragen.

Man erkennt, daß bei höheren Wasseranteilen bestimmte Dielektrizitätswerte bei höheren Leitfähigkeiten gemessen werden. Empirisch ermittelte Kurvenscharen der in Fig. 1 dargestellten Art ermöglichen bei einer kombinierten Messung von Leitfähigkeit und Dielektrizitätszahl eine Korrektur der Dielektrizitätsmessung durch die Leitwertbestimmung.

Durch die Kenntnis des Leitwertes können nun die Quereinflüsse auf die Kapazität durch Verunreinigungen des Kraftstoffes auskorrigiert werden. Wie Fig. 1 zeigt, wirkt beispielsweise ein höherer Wasseranteil im Kraftstoff kapazitätserhöhend. Durch Messung des Leitwertes kann diese Erhöhung der Kapazität bei der Bestimmung des Alkoholanteils berücksichtigt werden.

Bei der Auswertung wird die auf die Länge bezogene Leitfähigkeit als G′ bezeichnet, während die auf die Länge bezogene Kapazität, die wiederum von der Dielektrizitätskonstanten abhängt, als C′ bezeichnet wird. Dann ergibt sich z.B. für den Wellenwiderstand:

$$Z_L = (R′ + j\omega L′)^{1/2} * (G′ + j\omega C′)^{-1/2}$$

wobei R′ den von der Leitung gebildeten Widerstandsbelag und L′ ein Maß für die Induktivität pro Längeneinheit darstellt.

Das Dämpfungsmaß $\alpha$ und das Phasenmaß $\beta$ berechnen sich gemäß der Formel:

$$\alpha + j\beta = ((R′ + j\omega L′) * (G′ + j\omega C′))^{1/2}$$

Die Ausbreitungsgeschwindigkeit (Phasengeschwindigkeit) berechnet sich zu:

$$v = (L′C′)^{-1/2}$$

Gemäß einer bevorzugten Ausführungsform wird als Ausbreitungsparameter die Ausbreitungsgeschwindigkeit (Phasengeschwindigkeit) einer elektromagnetischen Welle bestimmt, die in starkem Maße von der Dielektrizitätskonstante des Mediums, mit dem der Wellenleiter gefüllt ist, abhängt. Somit kann aus der Ausbreitungsgeschwindigkeit das Mischungsverhältnis bzw. der Heizwert rechnerisch oder schaltungstechnisch bestimmt werden.

Besondere Vorteile sind erreichbar, wenn die Feststellung der Ausbreitungsgeschwindigkeit durch Messung der Laufzeit oder der Laufzeitdifferenz zwischen zwei oder mehreren Punkten erfolgt. Dabei braucht auch nicht wirklich die Bestimmung der Ausbreitungsgeschwindigkeit als solcher zu erfolgen, sondern die Laufzeit bzw. Laufzeitdifferenz kann unmittelbar rechnerisch oder schaltungstechnisch zur Bestimmung des Alkoholgehaltes oder des Heizwertes dienen.

Eine bevorzugte Ausführungsform einer solchen Schaltung ist in Fig. 2 wiedergegeben. Als Wellenleiter dient eine vom Kraftstoff durchströmte Leitung 3 mit metallischer Umhüllung, in die ein Innenleiter 4 eingebaut ist, der in bestimmten Abständen durch Zentrierscheiben 5 in der Mitte gehalten wird.

Ein Impulsgenerator 6 erzeugt einen Rechteckimpuls, der an Eingang 7 in den durch die Kraftstoffleitung 3 gebildeten Wellenleiter eingekoppelt wird. Am Ausgang 8 wird der Impuls wieder ausgekoppelt und mit dem über Leitung 9 zugeführten Originalimpuls in einer Auswerteschaltung 10 verglichen. Die Laufzeitdifferenz stellt ein Maß für die Wellenausbreitungsgeschwindigkeit dar und variiert entsprechend der Zusammensetzung des Kraftstoffes. In einem nachfolgenden Auswerterechner kann dann hieraus der Alkoholgehalt bzw. der Heizwert bestimmt werden.

Die beschriebene Ausführungsform stellt nur eine Variante von vielen Möglichkeiten darin so kann beispielsweise auch durch einen Wellenwiderstandssprung im Wellenleiter (z.B. des Innenleiters) eine Reflektion des Impulses verursacht werden. Am Eingang 7 kommt der reflektierte Impuls nach der doppelten Laufzeit, die er für eine Strecke benötigen würde, wieder an. Diese Laufzeit kann dann unmittelbar am Eingang 7 bestimmt werden, so daß die zusätzliche Leitung 9 entfallen kann.

In einer modifizierten Ausführungsform kann auch auf den Einbau des Innenleiters 4 verzichtet werden, wenn mit ausreichend hohen Frequenzen gearbeitet wird, die jenseits der sogenannten Cut-Off-Frequenz des durch den dann von der Kraftstoffleitung gebildeten Hohlleiters liegen.

Eine weitere vorteilhafte Möglichkeit ergibt sich gerade durch die Bestimmung der Cut-Off-Frequenz des durch die Kraftstoffleitung gebildeten Hohlleiters. Außer von den geometrischen Eigenschaften der Leitung hängt diese Cut-Off-Frequenz, oberhalb derer keine Wellenausbreitung mehr stattfindet, von den Stoffwerten - insbesondere der Dielektrizitätszahl - und damit der Zusammensetzung des Kraftstoffes ab, so daß auch die Cut-Off-Frequenz ein Maß für den Alkoholgehalt darstellt.

Die Bestimmung der Cut-Off-Frequenz kann wiederum auf verschiedene Arten erfolgen. Zwei bevorzugte Ausführungsformen sind in Fig. 3 dargestellt. Ein durchstimmbarer Frequenzgenerator 31 speist einen mit Kraftstoff gefüllten Wellenleiter 32 am Einspeisungspunkt 33. Am Auskopplungspunkt 34 ist ein Detektor 35 angeschlossen, der feststellt, ob an diesem Punkt noch ein Signal detektiert werden kann. Die Frequenz, oberhalb der kein Signal mehr detektiert werden kann, stellt dann das Maß für den Alkoholgehalt bzw. den Heizwert dar. Eine zweite Ausführungsform benutzt einen am Einspeisungspunkt 33 angebrachten Detektor 36, der durch Messung der Spannung oder des Stromes am Einspeisungspunkt 33 feststellen kann, ob die eingespeiste Frequenz durch den Wellenleiter transportiert wird. Jede Frequenz oberhalb der Cut-Off-Frequenz wird nicht transportiert, sondern vielmehr am Einspeisungspunkt 33 reflektiert, woraus sich am Einspeisungspunkt 33 andere Strom- und Spannungsverhältnisse ergeben. Dies wird durch den Detektor 36 festgestellt. Die Frequenz, ab der sich die Spannungsverhältnisse ändern (Spannungsvergrößerung), stellt ein Maß für den Alkoholgehalt bzw. den Heizwert dar.

Schließt man einen Wellenleiter an beiden Enden nicht mit dem Wellenwiderstand des Wellenleiters ab, erhält man einen Resonator, dessen Resonanzfrequenzen unter anderem direkt von der Phasengeschwindigkeit der Welle im Wellenleiter abhängen. So kann sehr vorteilhaft ein Teil des Kraftstoffsystems als Resonator ausgebildet werden, bei dem die Messung der Resonanzfrequenz zur Bestimmung der Dielektrizitätszahl des in dem Wellenleiter vorhandenen Mediums, also z.B. des Kraftstoffs, herangezogen werden kann.

Besondere Vorteile erreicht man bei Verwendung von bereits vorhandenen Teilen des Kraftstoffsystems, wie z.B. der Kraftstoffgalerie. Diese Komponente des Kraftstoffsystems wird meistens aus leitfähigem Material mit rechteckigem Querschnitt ausgebildet, das unmittelbar als sogenannter Hohlleiter oder auch als Hohlraumresonator verwendet werden kann.

Eine bevorzugte Ausführungsform zeigt Fig. 4. Hier wird zur Bestimmung einer Resonanzfrequenz des den Kraftstoff enthaltenden Resonators 41 ein aktives Hochfrequenzbauteil 42, z.B. ein Gunn-Element, eine Lawinen-Effekt-Diode oder auch eine Tunnel-Diode, verwendet. Diesem Bauteil wird durch den Resonator 41 eine bestimmte, von dem Füllungsmedium abhängige Resonanzfrequenz aufgeprägt, durch deren Messung in einer Schaltung 43 der Alkoholgehalt bzw. der Heizwert bestimmt werden kann. Der Resonator wird im Ausführungsbeispiel über Zuleitung 44 von Kraftstoff gespeist. Entsprechend den Pfeilen 45, 46, 47 und 48 erfolgt die Versorgung der Einspritzdüsen, und über Ableitung 49 verläßt ein Teil des Kraftstoffs den Resonator 41 wieder in Richtung eines Druckregelventils.

Zur Auswertung dieser relativ hohen Resonanzfrequenz kann diese durch Mischung mit einer zweiten Frequenz in einen der Auswertung besser zugänglichen Frequenzbereich heruntergesetzt werden.

Besondere Vorteile ergeben sich hinsichtlich Baugröße, Störempfindlichkeit, Störabstrahlung und Temperatureinfluß bei Integration der gesamten Meß- und Auswerteelektronik oder eines Teiles davon in das den Kraftstoff enthaltende System z.B. auch in den oben beschriebenen Hohlraumresonator.

Der Wellenwiderstand eines Wellenleiters wird u.a. auch durch die Stoffwerte des in dem Wellenleiter enthaltenen Stoffes bestimmt. So hat auch die Dielektrizitätszahl des Materials einen starken Einfluß auf den Wellenwiderstand. Durch Messung des Wellenwiderstandes eines mit Kraftstoff gefüllten Wellenleiters ist somit die Bestimmung dieser Größe und daraus wiederum die Ermittlung des Alkoholanteiles bzw. des Heizwertes möglich.

Eine bevorzugte Ausführungsform zur Bestimmung des Wellenwiderstandes macht sich die unterschiedliche Amplitude oder Phase des an einem Übergang auf einen bekannten Wellenwiderstand reflektierten Signals zunutze.

Eine weitere vorteilhafte Ausführungsform ermöglicht durch Messung von Amplitude und/oder Phase des Stromes und/oder der Spannung eine Bestimmung des Wellenwiderstandes des Wellenleiters.

Eine Kombination der gemessenen Ausbreitungsparameter, wie beispielsweise von Imaginär- und Realteil des Wellenwiderstandes und/oder von Laufzeiten und/oder Resonanzfrequenzen kann eine Erhöhung der Genauigkeit der Bestimmung von Alkoholgehalt oder Heizwert erfolgen, da hierdurch beispielsweise der negative Einfluß von Verunreinigungen auf die Messung kompensiert werden kann.

Eine bevorzugte Ausführungsform zur Bestimmung des dispersiven Verhaltens der Wellenleitung besteht darin, daß eine Leitung am Eingang mit einem Impuls bestimmter Form (z.B. rechteckförmig) gespeist wird. Durch das dispersive Verhalten der Leitung werden unterschiedliche Frequenzanteile mit verschiedenen Ausbreitungsgeschwindigkeiten auf der Leitung geführt. Dadurch kommt es zu einer Impulsverzerrung. Am Ende der Leitung wird nun die Impulsform analysiert und daraus Rückschlüsse auf die Dispersion des im Wellenleiter enthaltenen Mediums und somit auf das Mischungsverhältnis bzw. den Heizwert des Kraftstoffes rechnerisch oder schaltungstechnisch gezogen.

Eine weitere Verbesserung des Verfahrens zur Feststellung des Alkoholgehaltes und/oder des Heizwertes von Kraftstoffen kann dadurch erreicht werden, daß zusätzlich die Temperatur des Kraftstoffs bestimmt und zur Kompensation des Temperatureinflusses verwendet wird.

Bei Anwendung des Verfahrens gemäß der Erfindung auf die Steuerung bzw. Regelung von Einspritzbrennkraftmaschinen wird zweckmäßig so vorgegangen, daß die Messung des Alkoholanteils und/oder des Heizwertes des zugeführten Kraftstoffs zur Vorsteuerung der Einspritzmenge dient, während die Feinregulierung des Luftverhältnisses über eine Lambdaregelung bekannter Art erfolgt. Dabei werden weitere Vorteile erreicht, wenn die Meß- und/oder Auswerteschaltung in das Einspritzsystem schaltungs- und/oder programmtechnisch integriert wird.

## Patentansprüche

1. Verfahren zur Feststellung des Alkoholgehaltes und/oder des Heizwertes von Kraftstoffen über die Bestimmung von wenigstens einem Ausbreitungsparameter von elektromagnetischen Wellen, wobei an einem wenigstens teilweise von Kraftstoff durchströmten Wellenleiter wenigstens ein zeitbezogener Ausbreitungsparameter der elektromagnetischen Wellen gemessen, daß zusätzlich die Temperatur des durchströmenden Kraftstoffs gemessen wird, und die gemessenen Werte in einer Auswerteschaltung als Maß für den Alkoholgehalt und/oder den Heizwert des Kraftstoffs ausgewertet werden, wobei die gemessene Temperatur des Kraftstoffs zur Kompensation des Temperatureinflusses verwendet wird.

2. Verfahren nach Anspruch ein, dadurch gekennzeichnet, daß in dem von Kraftstoff durchströmten Wellenleiter die Laufzeit, zumindest die Laufzeitdifferenz einer elektromagnetischen Welle zwischen wenigstens zwei Punkten bestimmt und rechnerisch und/oder schaltungstechnisch als Maß für den Alkoholgehalt und/oder den Heizwert des Kraftstoffs ausgewertet wird.

3. Verfahren nach Anspruch 1 oder 2, dadurch gekennzeichnet, daß die Laufzeit einer reflektierten Welle als Maß für den Alkoholgehalt und/oder den Heizwert des Kraftstoffs ausgewertet wird.

4. Verfahren nach Anspruch 1, dadurch gekennzeichnet, daß die Cut-Off-Frequenz des vom Kraftstoff durchströmten Wellenleiters bestimmt und entweder für sich oder durch Verknüpfung mit anderen Größen als Maß für den Alkoholgehalt und/oder den Heizwert des Kraftstoffs ausgewertet wird.

5. Verfahren nach einem der Ansprüche 1 - 4, dadurch gekennzeichnet, daß wenigstens eine Resonanzfrequenz eines von Kraftstoff durchströmten und als Resonator ausgebildeten Wellenleiters bestimmt und schaltungstechnisch oder rechnerisch allein oder zusammen mit anderen Größen als Maß für den Alkoholgehalt und/oder den Heizwert des Kraftstoffs ausgewertet wird.

6. Verfahren nach Anspruch 5, dadurch gekennzeichnet, daß die Bestimmung der Resonanzfrequenzen mit Hilfe eines aktiven Hochfrequenzbauteiles erfolgt, das in den vom Kraftstoff durchströmten Wellenleiter integriert ist, wobei die jeweils entstehenden Resonanzen gemessen und rechnerisch oder schaltungstechnisch als Maß für den Alkoholgehalt und/oder den Heizwert des Kraftstoffs ausgewertet werden.

7. Verfahren nach einem der Ansprüche 1 - 6, dadurch gekennzeichnet, daß zur Erhöhung der Genauigkeit der Messung und/oder zur Kompensation des Einflusses von Verunreinigungen und/oder Zusätzen im Kraftstoff wenigstens ein zusätzlicher, nicht zeitbezogener Ausbreitungsparameter kombiniert gemessen bzw. ausgewertet werden.

8. Verfahren zur Feststellung des Alkoholgehalts und/oder des Heizwerts von Kraftstoffen über die Bestimmung von wenigstens einem Ausbreitungsparameter von elektromagnetischen Wellen nach den voraufgegangenen Ansprüchen, insbesondere nach Anspruch 1, dadurch gekennzeichnet, daß der Wellenwiderstand eines vom Kraftstoff durchströmten Wellenleiters bestimmt und allein oder zusammen mit anderen Größen als Maß für den Alkoholgehalt und/oder den Heizwert des Kraftstoffs ausgewertet wird.

9. Verfahren zur Feststellung des Alkoholgehalts und/oder des Heizwerts von Kraftstoffen über die Bestimmung von wenigstens einem Ausbreitungsparameter von elektromagnetischen Wellen nach den voraufgegangenen Ansprüchen, insbesondere nach Anspruch 1, dadurch gekennzeichnet, daß der dispersive Einfluß eines von Kraftstoff in einem durchströmten und mit elektromagnetischen Wellen beaufschlagten Wellenleiters zur Messung des Alkoholgehalts bzw. des Heizwerts gemessen und ausgewertet wird.

10. Verfahren nach einem der Ansprüche 1 - 9, dadurch gekennzeichnet, daß als Wellenleiter Originalteile oder modifizierte Teile dienen, die im Kraftstoffsystem einer Brennkraftmaschine vorhanden sind.

11. Verfahren nach einem der Ansprüche 1 - 10, dadurch gekennzeichnet, daß die Meßschaltung oder Teile davon ganz oder teilweise in den Wellenleiter oder Resonator integriert werden.

12. Verfahren nach einem der Ansprüche 1 - 11, dadurch gekennzeichnet, daß bei Anwendung auf die Steuerung bzw. Regelung von Einspritzbrennkraftmaschinen die Messung des Alkoholanteils und/oder des Heizwerts des zugeführten Kraftstoffs zur Vorsteuerung der Einspritzmenge dient, während die Feinregulierung des Luftverhältnisses über eine Lambdaregeleung bekannter Art erfolgt.

13. Verfahren nach einem der Ansprüche 1 - 12, dadurch gekennzeichnet, daß bei der Anwendung auf Einspritzbrennkraftmaschinen die Meß- und/oder Auswerteschaltung in das Einspritzsystem schaltungs- und/oder programmtechnisch integriert wird.

## Claims

1. A method for determining the alcohol content and/or the calorific value of fuels by determining at least one propagation parameter of electromagnetic waves, wherein at least one time-related propagation parameter of the electromagnetic waves is measured in a waveguide through which fuel flows at least in part, that additionally the temperature of the fuel flowing through is measured, and the measured values are evaluated in an evaluation circuit as a measurement of the alcohol content and/or the calorific value of the fuel, with the measured temperature of the fuel being used to compensate for the effect of temperature.

2. A method according to Claim 1, characterised in that the transit time, or at least the transit-time difference between at least two points, of an electromagnetic wave in the waveguide through which fuel flows, is determined and is evaluated computationally and/or by means of the circuit design as a measurement of the alcohol content and/or the calorific value of the fuel.

3. A method according to C1laim 1 or 2, characterised in that the transit time of a reflected wave is evaluated as a measurement of the alcohol content and/or the calorific value of the fuel.

4. A method according to Claim 1, characterised in that the cut-off frequency of the waveguide through which fuel flows is determined and is evaluated either alone or by linking with other variables as a measurement of the alcohol content and/or the calorific value of the fuel.

5. A method according to one of Claims 1 to 4, characterised in that at least one resonance frequency of a waveguide through which fuel flows and which is designed as a resonator is determined and is evaluated on its own or together with other variables as a measurement of the alcohol content and/or the calorific value of the fuel by means of the circuit design or computationally.

6. A method according to Claim 5, characterised in that the determination of the resonance frequencies is effected with the aid of an active high-frequency component which is integrated in the waveguide through which fuel flows, with the resonances produced each time being measured and being evaluated computationally or by means of the circuit design as a measurement of the alcohol content and/or the calorific value of the fuel.

7. A method according to one of Claims 1 to 6, characterised in that in order to increase the accuracy of the measurement and/or to compensate for the influence of impurities and/or additives in the fuel, at least one additional, non-time-related propagation parameter is measured or evaluated in combination.

8. A method for determining the alcohol content and/or the calorific value of fuels by means of the determination of at least one propagation parameter of electromagnetic waves according to the preceding claims, in particular according to Claim 1, characterised in that the wave impedance of a waveguide through which fuel flows is determined and is evaluated alone or together with other variables as a measurement of the alcohol content and/or the calorific value of the fuel.

9. A method for determining the alcohol content and/or the calorific value of fuels by means of the determination of at least one propagation parameter of electromagnetic waves according to the preceding claims, in particular according to Claim 1, characterised in that the dispersive effect of a waveguide through which fuel flows and which is acted upon by electromagnetic waves is measured and evaluated for measuring the alcohol content or the calorific value.

10. A method according to one of Claims 1 to 9, characterised in that original parts or modified parts which are present in the fuel system of an internal combustion engine serve as waveguides.

11. A method according to one of Claims 1 to 10, characterised in that the measuring circuit or parts thereof are integrated entirely or in part in the waveguide or resonator.

12. A method according to one of Claims 1 to 11, characterised in that for application to the control or regulation of fuel-injection internal combustion engines, the measurement of the alcohol content and/or of the calorific value of the fuel supplied serves for preliminary control of the injection quantity, whereas the fine control of the air ratio takes place by means of a lambda control of known type.

13. A method according to one of Claims 1 to 12, characterised in that when applied to fuel-injection internal combustion engines the measuring and/or evaluation circuit is integrated into the fuel-injection system by means of the circuit and/or program design.

**Revendications**

1. Procédé pour déterminer la teneur en alcool et/ou la valeur calorifique de carburants par l'intermédiaire de la détermination d'au moins un paramètre de propagation d'ondes électromagnétiques, dans lequel on mesure au moins un paramètre de propagation des ondes électromagnétiques rapporté au temps sur au moins un guide d'ondes qui est parcouru par du carburant, du moins partiellement, on mesure en supplément la température du carburant qui circule et on exploite les valeurs mesurées dans un circuit d'exploitation pour servir de mesure de la teneur en alcool et/ou de la valeur calorifique du carburant, la température mesurée du carburant étant utilisée pour compenser l'influence de la température.

2. Procédé selon la revendication 1, caractérisé par le fait que, dans le guide d'ondes parcouru par le carburant, on détermine la durée de parcours d'une onde électromagnétique, ou du moins la différence entre ces durées de parcours entre deux points au moins, et un l'exploite par le calcul et/ou par la technique des circuits pour servir de mesure de la teneur en alcool et/ou de la valeur calorifique du carburant.

3. Procédé selon la revendication 1 ou 2, caractérisé par le fait que l'on exploite la durée de parcours d'une onde réfléchie pour servir de mesure de la teneur en alcool et/ou de la valeur calorifique du carburant.

4. Procédé selon la revendication 1, caractérisé par le fait que l'on détermine la fréquence de coupure du guide d'ondes parcouru par le carburant et qu'on l'exploite, soit pour elle-même, soit en combinaison avec d'autres grandeurs, pour servir de mesure de la teneur en alcool et/ou de la valeur calorifique du carburant.

5. Procédé selon l'une des revendications 1 à 4, caractérisé par le fait que l'on détermine au moins une fréquence de résonance d'un guide d'ondes qui est parcouru par le carburant et qui est réalisé sous la forme d'un résonateur, et qu'on l'exploite par la technique des circuits ou par le calcul, soit seule, soit en combinaison avec d'autres grandeurs, pour servir de mesure de la teneur en alcool et/ou de la valeur calorifique du carburant.

6. Procédé selon la revendication 5, caractérisé par le fait que la détermination des fréquences de résonance a lieu à l'aide d'un composant actif à haute fréquence qui est intégré au guide d'ondes parcouru par le carburant, cependant que l'on mesure les résonances qui se produisent à chaque fois et qu'on les exploite par le calcul ou par la technique des circuits pour servir de mesure de la teneur en alcool et/ou de la valeur calorifique du carburant.

7. Procédé selon l'une des revendications 1 à 6, caractérisé par le fait que l'on mesure ou, respectivement, que l'on exploite en combinaison un paramètre de propagation supplémentaire qui n'est pas rapporté au temps, et ce, en vue d'augmenter la précision de la mesure et/ou de compenser l'influence d'impuretés et/ou d'additifs contenus dans le carburant.

8. Procédé pour déterminer la teneur en alcool et/ou la valeur calorifique de carburants par l'intermédiaire de la détermination d'au moins un paramètre de propagation d'ondes électromagnétiques selon les revendications précédentes, et en particulier selon la revendication 1, caractérisé par le fait que l'on détermine l'impédance caractéristique d'un guide d'ondes parcouru par le carburant, et qu'on l'exploite seule ou en combinaison avec d'autres grandeurs pour servir de mesure de la teneur en alcool et/au de la valeur calorifique du carburant.

9. Procédé pour déterminer la teneur en alcool et/ou la valeur calorifique de carburants par l'intermédiaire de la détermination d'au moins un paramètre de propagation d'ondes électromagnétiques selon les revendications précédentes, et en particulier selon la revendication 1, caractérisé par le fait que, pour mesurer la teneur en alcool ou la valeur calorifique, respectivement, on mesure et on exploite l'influence sur la dispersion d'un guide d'ondes qui est parcouru par du carburant et qui est soumis à l'action d'ondes électromagnétiques.

10. Procédé selon l'une des revendications 1 à 9, caractérisé par le fait que l'on utilise comme guide d'ondes des parties d'origine ou des parties modifiées qui sont prémentes dans le système d'amenée du carburant d'un moteur à combustion interne.

11. Procédé selon l'une des revendications 1 à 10, caractérisé par le fait que le circuit de mesure ou des parties de celui-ci sont intégrées totalement ou partiellement au guide d'ondes ou au résonateur.

12. Procédé selon l'une des revendications 1 à 11, caractérisé par le fait que, dans le cas de son utilisation pour la commande ou, respectivement, pour la régulation de moteurs à combustion interne à injection, la mesure de la teneur en alcool et/ou de la valeur calorifique du carburant qui est amené sert à la commande préalable de la quantité injectée, tandis que la régulation fine du rapport air/carburant a lieu par l'intermédiaire d'une régulation lambda d'un genre connu.

13. Procédé selon l'une des revendications 1 à 12, caractérisé par le fait que, dans le cas de l'utilisation pour des moteurs à combustion interne à injection, le circuit de mesure et/ou d'exploitation est intégré au système d'injection quant aux techniques des circuits et/ou des programmes.

FIG. 1

FIG. 4

FIG. 3

FIG. 2